# EUROPÄISCHE PATENTSCHRIFT

(11) **EP 0 574 701 B1**
(45) Veröffentlichungstag und Bekanntmachung des Hinweises auf die Patenterteilung: **10.07.1996**
(21) Anmeldenummer: 93107739.0
(22) Anmeldetag: 12.05.1993
(51) Int. Cl.: A61B 17/16, A61L 31/00, A61M 1/00

(54) **Bearbeitungswerkzeug für Knochen**
Working tool for bones
Outil de traivail pour les os

(30) Priorität: 17.06.1992 CH 1908/92
(43) Veröffentlichungstag der Anmeldung: 22.12.1993
(73) Patentinhaber: Kropf, Philipp Rolf, CH-8142 Uitikon (CH)
(72) Erfinder: Kropf, Philipp Rolf, CH-8142 Uitikon (CH)
(74) Vertreter: Blum, Rudolf Emil Ernst

(56) Entgegenhaltungen:
- EP-A- 0 142 378
- EP-A- 0 303 810
- DE-A- 2 457 713
- US-A- 4 830 000
- US-A- 5 100 267

## Beschreibung

Die Erfindung betrifft ein Bearbeitungswerkzeug für Knochen, mit einem mit mindestens einer Schneide versehenem Bearbeitungsteil zum Abtragen von Knochen durch Drehung des Bearbeitungswerkzeuges und mit einem Adapterteil, das zur Koppelung des Bearbeitungswerkzeuges an ein in Drehung versetzbares Antriebswerkzeug bestimmt ist. Ein derartiges Bearbeitungswerkzeug für Knochen ist beispielsweise aus der US-A-5 100 267 bekannt. Bei dem bekannten Bearbeitungswerkzeug sind die Schneiden aus Metall.

Zur Bearbeitung der Lagerflächen von Knochen zur Aufnahme eines künstlichen Gelenkteiles, insbesondere von künstlichen Gelenkpfannen, werden bekannterweise drehend angetriebene Fräser, Raffelfräser und Raffeln aus Metall oder Metallegierungen verwendet, z.B. solche aus Stahl, rostfreiem Stahl, oberflächenbehandeltem Stahl, chemisch vernickeltem, oder hartverchromtem Stahl. Solche Fräser oder Raffeln sind in verschiedenen Grössen und Formen bekannt. Diese Bearbeitungswerkzeuge werden nach jeder Operation gereinigt und mittels Sterilisationsgeräten wiederum steril aufbereitet. Das Wiederaufbereiten ist in bezug auf das Kontaminationsrisiko problematisch. So sind die Fräser oder Raffeln nach der Operation mit Blut und mit Knochensubstanz behaftet und müssen z.B. mit Bürsten von Hand gereinigt werden. Diese Arbeit ist unangenehm und durch die scharfen Schneiden der Fräser oder Raffeln besteht Verletzungsgefahr. Bei der Handhabung dieser Bearbeitungswerkzeuge bei der Sterilisation und der Reinigung besteht ferner die Gefahr, dass die Werkzeuge mit anderen Metallteilen in Berührung kommen und dadurch stumpf werden. Eine geringe Abstumpfung ergibt sich natürlich auch durch die mehrfache Verwendung dieser Werkzeuge trotz deren Ausbildung aus sehr harten metallischen Werkstoffen. Auch eine geringfügige Stumpfheit der Schneiden wirkt sich indes beim Gebrauch nachteilig aus. Ferner können die bekannten Werkzeuge aus Metall - je nach Ausführung - sehr schwer sein, was deren Handhabung bei der Operation erschwert.

Das Problem der Reinigung stellt sich auch bei Knochenbohrern, welche ebenfalls in der Form von Fräsern ausgeführt sein können, oder in der Form von Spiralbohrern mit einer spiralförmigen Schneide. Die genannten Metallwerkzeuge sind ferner aufwendig in der Herstellung und entsprechend teuer.

Der Erfindung liegt deshalb die Aufgabe zugrunde, ein Bearbeitungswerkzeug zu schaffen, welches die genannten Nachteile nicht aufweist.

Dies wird bei einem Bearbeitungswerkzeug der eingangs genannten Art dadurch erreicht, dass der Bearbeitungsteil mit der mindestens einen Schneide aus Kunststoff besteht.

Das Bearbeitungswerkzeug aus Kunststoff ist sehr kostengünstig als nur einmal zu verwendendes Werkzeug herstellbar. Dadurch entfällt das Problem der Reinigung und Sterilisation gänzlich; ebenfalls ist bei jeder Knochenbearbeitung ein optimal scharfes Werkzeug sichergestellt.

Vorzugsweise besteht auch das Adapterteil des Bearbeitungswerkzeuges aus Kunststoff und das Bearbeitungswerkzeug wird als bereits steriles Werkzeug in einer die Sterilität erhaltenden Verpackung dem Gebrauch zugeführt.

Im folgenden werden Ausführungsbeispiele der Erfindung anhand der Figuren näher erläutert. Dabei zeigt
Figur 1 einen als Hohlkörper ausgeführten Raffelfräser in Seitenansicht;
Figur 2 den Raffelfräser von Figur 1 in der Draufsicht;
Figur 3 einen als Hohlkörper ausgebildeten Fräser ohne Adapterteil in der Seitenansicht;
Figur 4 den Fräser von Figur 3 in der Draufsicht;
Figur 5 einen Vorfräser ohne Adapterteil in der Seitenansicht;
Figur 6 den Vorfräser von Figur 5 in Draufsicht;
Figur 7 einen als Vollkörper ausgeführten Fräser ohne Adapterteil in der Seitenansicht;
Figur 8 den Fräser von Figur 7 in der Draufsicht;
Figur 9 schematisch einen Raffelfräser in Schnittansicht mit einem Hohlkörper als Bearbeitungsteil und mit einer Zufuhr für Spülflüssigkeit und einer Wegfuhr für Bearbeitungsrückstände;
Figur 10 schematisch einen Raffelfräser in Schnittansicht mit einem Hohlkörper als Bearbeitungsteil ähnlich Figur 9 und anders gestalteter Zuführ- und Wegführmittel;
Figur 11 einen Spiralbohrer in Seitenansicht; und
Figur 12 den Spiralbohrer von Figur 11 in Draufsicht.

Drehende, mit einer oder mehreren Schneiden materialabtragende Knochenbearbeitungswerkzeuge, welche an ein drehendes Antriebswerkzeug angekoppelt werden, sind in vielfältigen Formen als Bohrer, z.B. als Spiralbohrer, Fräser, Vorfräser, Raffelfräser und Raffeln aus Metall und Metall-Legierungen bekannt. Insbesondere werden solche Werkzeuge zur Bearbeitung der Lagerflächen von Knochen zur Aufnahme eines künstlichen Gelenkteiles verwendet, insbesondere zur Bearbeitung der Lagerflächen für künstliche Gelenkpfannen.

Beim Einsetzen einer Prothese ist das Säubern des Knochens von zentraler Bedeutung. So werden Knochenlametten freigeschabt, ausgekratzt, aber auch Bindegewebe oder Zementrückstände und andere Strukturen aus einem eventuellen Prothesenwechsel abgetragen. Eventuell wird auch nekrotisches Knochengewebe abgetragen.

Die Fräser, Raffelfräser und Raffeln und auch Bohrer werden nach jeder Operation gereinigt und mittels Dampfsterilisationsgeräten (Autoklaven) oder Heissluftsterilisationsgeräten wiederum steril aufbereitet. Dieses Wiederaufbereiten ist in bezug auf das Kontaminationsrisiko problematisch. So sind die Werkzeuge nach der Operation mit Blut und Knochen behaftet und müssen mit Bürsten manuell gereinigt werden. Durch die geschliffenen oder gehauenen scharfen Kanten besteht Verletzungsgefahr. Durch die mehrfache Verwendung sowie auch durch das Wiederaufbereiten dieser Metallteile, werden die einst scharfen Schneideöffnungen stumpf und erfüllen ihren Zweck nicht mehr optimal, oder die Adapter sind beschädigt, so dass ein wiederholtes Verwenden nicht mehr möglich ist.

Gemäss der Erfindung besteht nun der Bearbeitungsteil und vorzugsweise auch der Adapterteil des Bearbeitungswerkzeuges aus Kunststoff. Dies erlaubt die kostengünstige Herstellung eines nur einmal zu verwendenden Bearbeitungswerkzeuges, welches vorzugsweise bereits steril verpackt geliefert wird. Dies löst alle geschilderten Probleme der bekannten mehrfach zu verwendenden Werkzeuge aus Metall; das Kunststoffwerkzeug ist zu Beginn der Operation optimal scharf, da es neu und ungebraucht ist, und es ist steril. Nach der Operation wird es entsorgt; eine Reinigung und Sterilisation entfällt.

Als Kunststoffmaterial für die Werkzeuge kommen verschiedene Kunststoffe, (auch faserverstärkte Kunststoffe, mit Kohlefasern oder Glasfasern) infrage, z.B. POM Polyoxymethylen, PA Polyamid, PE Polyäthylen etc. Besonders geeignet ist z.B. Polycarbonat, insbesondere Polycarbonat vom Typ Macrolon 2805 der Firma Bayer.

Das Formen der nachfolgend beispielshalber gezeigten Raffeln und Fräser und auch aller anderen, nicht gezeigten Formen, kann jeweils durch eine bekannte, dem jeweiligen Kunststoff adäquate Technologie erfolgen, z.B. durch Spritzgiessen, Warmumformung oder mechanische Bearbeitung eines Rohlings.

Die Erfindung schlägt einen neuen Weg bei der Schaffung von drehenden Knochenbearbeitungswerkzeugen ein. Anstelle der Verwendung von sehr harten metallischen Werkstoffen zur Erhöhung der Standzeit wird ein im Vergleich weicher Werkstoff (Kunststoff) verwendet; dies ergibt aber überraschenderweise in der Praxis auf dem Anwendungsgebiet der Knochenbearbeitung keine Nachteile, sondern die bereits geschilderten Vorteile.

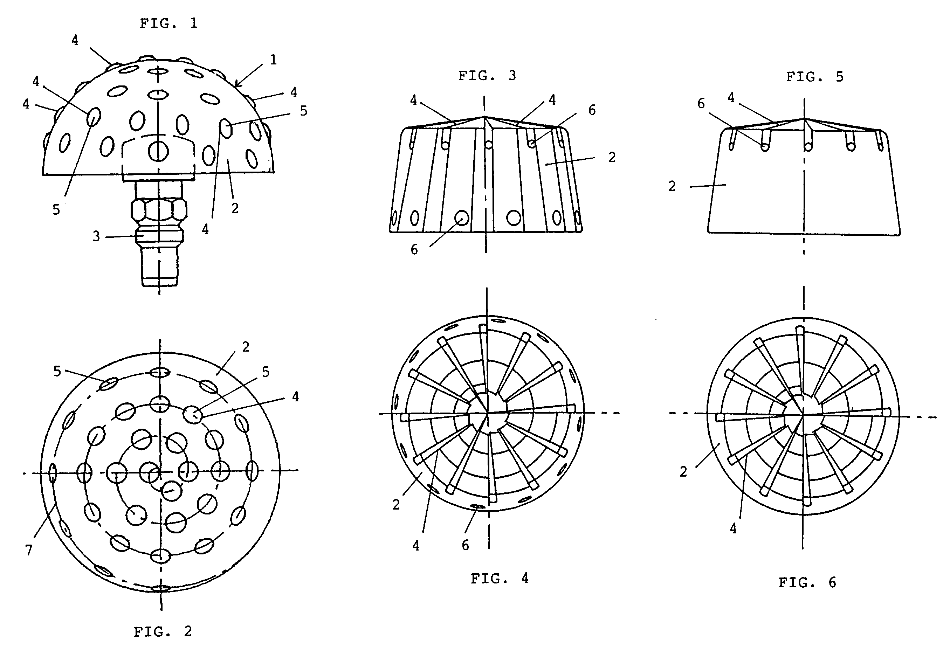

Figur 1 zeigt ein Bearbeitungswerkzeug 1 bekannter Form. Dieses weist einen Bearbeitungsteil 2 und einen Adapterteil 3 auf. Der Adapterteil 3 dient zur Kopplung des Bearbeitungswerkzeuges an ein Antriebswerkzeug, das die drehende Bewegung des Bearbeitungswerkzeuges 1 bewirkt. Das Antriebswerkzeug kann einen elektrischen, hydraulischen oder pneumatischen Antrieb umfassen. Das Antriebswerkzeug wird - da in verschiedenen Ausführungen bekannt - hier nicht näher erläutert. Der Adapterteil 3 ist derart ausgestaltet, dass das Bearbeitungswerkzeug mit einer entsprechenden Aufnahme am Antriebswerkzeug gekoppelt werden kann. Es sind verschiedene Formen solcher Adapterteile bekannt. In Figur 1 ist ein solches Adapterteil deshalb nur beispielsweise dargestellt. Es können für das Adapterteil auch beliebige andere bekannte Formen, jeweils abhängig von der Aufnahme des Antriebswerkzeuges vorgesehen sein. Bevorzugterweise besteht der Adapterteil 3 ebenfalls vollständig aus Kunststoff. Der Bearbeitungsteil 2 ist in dem gezeigten Beispiel als halbkugelförmiger Hohlkörper ausgestaltet. Auch andere Formen als die hemisphärische Form sind bekannt und können vorgesehen sein, z.B. eine konische oder eine parabolische Form. Bei der bekannten Ausführung des Bearbeitungsteiles aus Metall sind die Schneiden zum Abtragen des Knochens derart gebildet, dass im Hohlkörper eine Vielzahl von Ausnehmungen vorgesehen sind, deren Rand jeweils bereichsweise über die Aussenkontur der Halbkugel vorsteht, um jeweils eine löffelförmige Schneide zu bilden. Der in Figur 1 gezeigte Bearbeitungsteil 2, der nun erfindungsgemäss aus Kunststoff geformt ist, weist die selbe Ausgestaltung der Schneiden auf. Das heisst, es sind eine Vielzahl von Ausnehmungen 5 in der Halbkugel vorgesehen, deren Rand jeweils teilweise über die Aussenfläche der Halbkugel vorsteht, um eine Vielzahl von Schneiden 4 zu bilden. Wie aus der Draufsicht von Figur 2 ersichtlich, sind die Ausnehmungen 5 entlang einer Spirallinie auf der Halbkugel angeordnet. In Figur 2 ist dies zeichnerisch durch die Linie 7 verdeutlicht. Solche Raffelfräser wie in Figur 1 und 2 gezeigt, werden jeweils als ganze Sätze von verschiedenen Durchmessern von z.B. 10 mm bis 80 mm angeboten. Die Ausgestaltung solcher Raffelfräser als Hohlkörper erlaubt die Aufnahme des abgetragenen Materials im Hohlkörperinneren.

Die Figuren 3 und 4 zeigen den Bearbeitungsteil 2 eines weiteren Bearbeitungswerkzeuges. Das Adapterteil ist in diesen Figuren und ebenso in den Figuren 5 bis 8 nicht dargestellt. Der Fräser gemäss den Figuren 3 und 4 ist ebenfalls als Hohlkörper ausgestaltet. Bei diesem Fräser sind die Schneiden 4 als entlang von Mantellinien des Hohlkörpers verlaufende vorstehende Schneidkanten ausgebildet. Ebenfalls sind Durchbrüche 6 in der Wandung des Hohlkörpers vorgesehen, um den Eintritt von abgetragenem Material in den Hohlkörper hinein zu ermöglichen. Die Figuren 5 und 6 zeigen einen Vorfräser, der ähnlich wie der Fräser gemäss den Figuren 3 und 4 ausgebildet ist, welcher aber nur Schneidkanten 4 an seinem vorderen Bereich aufweist.

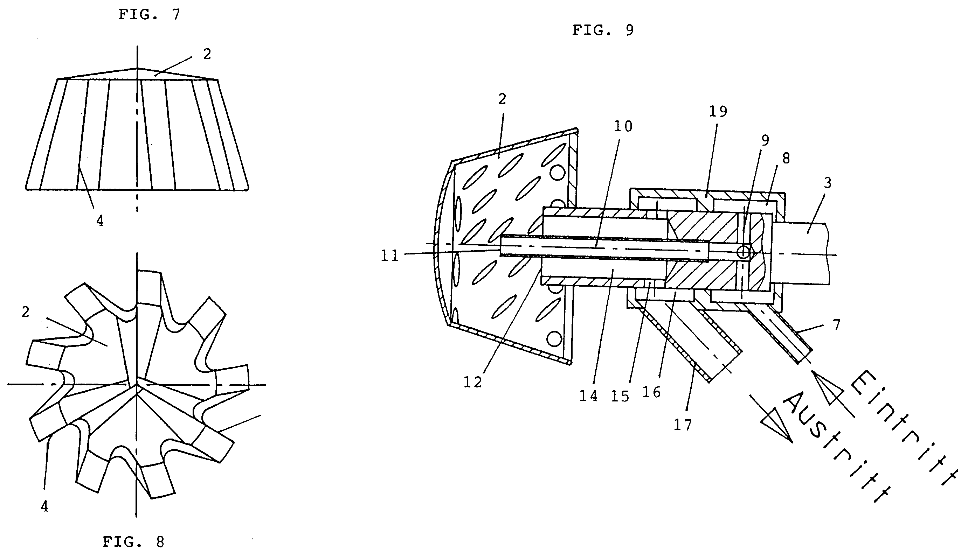

Die Figuren 7 und 8 zeigen einen Fräser, der als Vollkörper aus Kunststoff ausgebildet ist.

Figur 9 zeigt schematisch in einer Schnittansicht einen weiteren Aspekt der Erfindung. Dabei ist ein Bearbeitungswerkzeug mit einem Bearbeitungsteil 2 und einem teilweise dargestellten Adapterteil 3 gezeigt. Das Adapterteil weist nun Zuführmittel und Wegführmittel für eine Spülflüssigkeit bzw. für die Spülflüssigkeit und das abgetragene Material auf. Die Spülung des Bearbeitungsteils bzw. der bearbeiteten Knochenpartie bietet verschiedene Vorteile. Einerseits erfolgt durch die Spülflüssigkeit eine Kühlung des bearbeiteten Knochens, was eine Beschädigung des verbleibenden Knochenmaterials infolge grosser Temperatur durch die Bearbeitung verhindert. Andererseits wird durch die Spülflüssigkeit eine besonders gute Entfernung der Bearbeitungsrückstände aus der Bearbeitungszone erreicht. Bei dem in Figur 9 gezeigten Bearbeitungsteil 2, der als Hohlkörper ausgestaltet ist, erfolgt die Zufuhr und die Wegfuhr der Spülflüssigkeit durch das Innere des Hohlkörpers 2. Die Zufuhr der Spülflüssigkeit erfolgt dabei über ein Anschlussstück 7 am Adapter 3, an welchem Anschlussstück ein Schlauch angeschlossen werden kann, der Spülflüssigkeit von einer Quelle her zuführt. Vom Anschlussstück 7 gelangt die Spülflüssigkeit in eine Kammer 8 im Adapterteil und von dort über radiale Kanäle 9 in eine zentral angeordnete Zuleitung 10, welche die Spülflüssigkeit über eine Mündung 11 in das Innere des Hohlkörpers 2 abgibt. Von einer gegenüber der Mündung 11 versetzt angeordneten Ansaugöffnung 12 wird verunreinigte Spülflüssigkeit angesaugt und über einen zentralen Kanal 14 mit seitlichen Oeffnungen 15 in eine Kammer 16 abgegeben, aus welcher die verunreinigte Spülflüssigkeit in das Anschlussstück 17 gelangt, an welchem ein Schlauch angeschlossen ist, mittels welchem die verunreinigte Spülflüssigkeit weggeführt wird. Die Anschlussstücke 7 und 17 sind an einem feststehenden Halteteil 19 angeordnet, welches in sich ebenfalls die Kammern 8 und 16 bildet. Gegenüber dem feststehenden Halteteil 19 können die übrigen Teile des Adapters 3 die Drehbewegung für das Werkzeug ausführen. Die entsprechenden Lagerelemente und Abdichtungen sind in der schematischen Darstellung von Figur 9 nicht gezeigt.

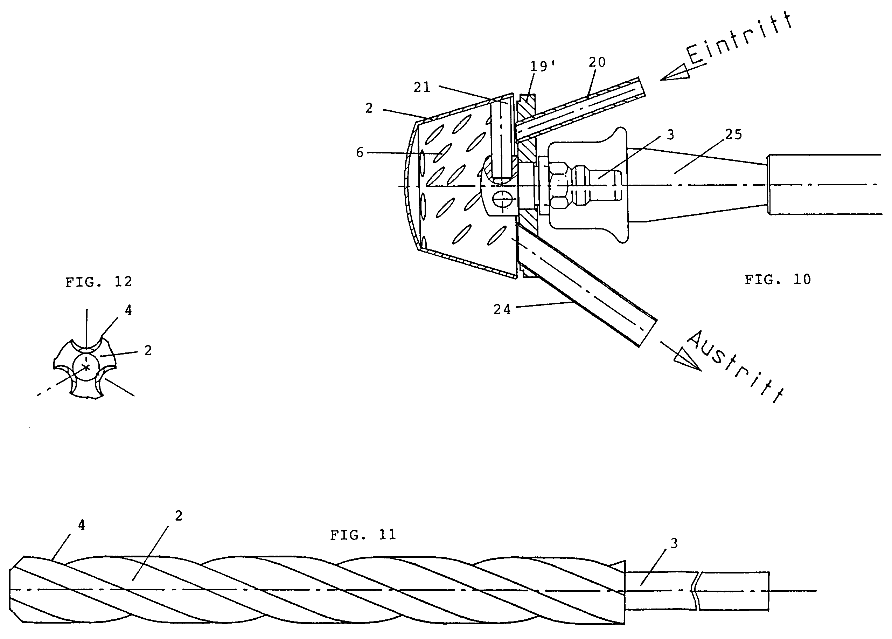

Figur 10 zeigt eine weitere Ausführungsform des Bearbeitungswerkzeugs mit Zufuhr und Wegfuhr von Spülflüssigkeit. In dieser Figur ist weiter ein Teil des Antriebswerkzeuges 25 dargestellt, in welchem der Adapterteil 3 befestigt ist. Die Zufuhr bzw. Wegfuhr der Spülflüssigkeit erfolgt hier über Anschlussstücke 20 bzw. 24, welche in einer gegenüber der Drehbewegung von Antriebswerkzeug, Adapterteil und Bearbeitungsteil feststehenden Halterung 19' gehalten sind. Die Einleitung der Spülflüssigkeit in den Hohlkörper 2 erfolgt durch das Anschlussstück 20. Mit dem Hohlkörper 2 rotierende Kanäle 21 sorgen für eine Verteilung der Spülflüssigkeit. Die verunreinigte Spülflüssigkeit wird durch das Anschlussstück 24 abgesaugt.

Das anhand von Fräsern gemäss Figur 9 und Figur 10 gezeigte Spülen der Bearbeitungszone kann auch bei Spiralbohrern aus Kunststoff ausgeführt werden. Diese Bohrer sind vorzugsweise mit einer zentral verlaufenden Bohrung zur Zufuhr und Wegfuhr von Spülflüssigkeit zu versehen. Seitliche Durchbrüche im Schaft des Bohrers ermöglichen den Zutritt der Spülflüssigkeit in die Bearbeitungszone bzw. deren Entfernung aus der Bearbeitungszone. Das Absaugen von Spülflüssigkeit, welche Knochenmaterial enthält, ermöglicht es, dieses Knochenmaterial durch entsprechende Bearbeitung der Spülflüssigkeit zurückzugewinnen. Das der Spülflüssigkeit entnommene Knochenmaterial kann im weiteren Verlauf einer Operation am Patienten wiederverwendet werden.

Figur 11 zeigt in Seitenansicht als Beispiel einen dreigängigen Spiralbohrer aus Kunststoff mit dem Bearbeitungsteil 2, mit den Schneiden 4 und mit dem Adapterteil 3. Figur 12 zeigt eine Draufsicht auf die Bohrerspitze des Bohrers von Figur 11.

## Patentansprüche

1. Bearbeitungswerkzeug (1) für Knochen, mit einem mit mindestens einer Schneide (4) versehenem Bearbeitungsteil (2) zum Abtragen von Knochen durch Drehung des Bearbeitungswerkzeuges und mit einem Adapterteil (3), das zur Koppelung des Bearbeitungswerkzeuges (1) an ein in Drehung versetzbares Antriebswerkzeug bestimmt ist, dadurch gekennzeichnet, dass der Bearbeitungsteil (2) mit der mindestens einen Schneide (4) aus Kunststoff besteht.

2. Bearbeitungswerkzeug nach Anspruch 1, dadurch gekennzeichnet, dass der Adapterteil (3) aus Kunststoff besteht.

3. Bearbeitungswerkzeug nach Anspruch 1 oder 2, dadurch gekennzeichnet, dass der Bearbeitungsteil (2) von einem Hohlkörper gebildet ist, dessen Aussenwandung mit einer Mehrzahl von Schneiden (4) und mit Oeffnungen (5,6) versehen ist.

4. Bearbeitungswerkzeug nach Anspruch 1 oder 2, dadurch gekennzeichnet, dass der Bearbeitungsteil (2) von einem Vollkörper gebildet ist, dessen Aussenwandung mit einer Mehrzahl von Schneiden (4) versehen ist.

5. Bearbeitungswerkzeug nach Anspruch 1 oder 2, dadurch gekennzeichnet, dass der Bearbeitungsteil einen langgestreckten Schaft mit mindestens einer spiralförmig den Schaft umlaufenden Schneide umfasst.

6. Bearbeitungswerkzeug nach Anspruch 5, dadurch gekennzeichnet, dass der Schaft mit mindestens einer Längsbohrung und mit seitlichen Oeffnungen versehen ist.

7. Bearbeitungswerkzeug nach einem der Ansprüche 1 bis 6, gekennzeichnet durch Zuführmittel (7,8, 9,10,11;20,21) für Spülflüssigkeit.

8. Bearbeitungswerkzeug nach einem der Ansprüche 1 bis 7, gekennzeichnet durch Wegführmittel (12, 14,15,16,17;24) zur Wegführung von Bearbeitungsrückständen.

9. Bearbeitungswerkzeug nach den Ansprüchen 3, 7 und 8 oder den Ansprüchen 6, 7 und 8, dadurch gekennzeichnet, dass die Zuführmittel zur Abgabe von Spülflüssigkeit in das Innere des Hohlkörpers bzw. des Schaftes ausgestaltet sind und dass die Wegführmittel zur Wegführung von mit Bearbeitungsrückständen verschmutzter Spülflüssigkeit aus dem Inneren des Hohlkörpers bzw. Schaftes ausgestaltet sind.

10. Bearbeitungswerkzeug nach einem der Ansprüche 1 bis 9, dadurch gekennzeichnet, dass das Bearbeitungswerkzeug steril ist und von einer die Sterilität aufrechterhaltenden Verpackungshülle umgeben ist.

## Claims

1. Tool (1) for machining bones, with a working part (2) that comprises at least one cutting edge (4) for abrading bone through rotation of the tool, and with an adapter (3) intended for coupling the tool (1) to a rotatable drive, characterized in that the working part (2) and its at least one cutting edge (4) is made of plastic.

2. Tool according to claim 1, characterized in that the adapter (3) is made of plastic.

3. Tool according to claim 1 or 2, characterized in that the working part (2) consists in a hollow body, the outer surface of which comprises several cutting edges (4) and apertures (5, 6).

4. Tool according to claim 1 or 2, characterized in that the working part (2) consists in a solid body, the outer surface of which comprises several cutting edges (4).

5. Tool according to claim 1 or 2, characterized in that the working part comprises an elongated shaft around which runs at least one spiral-shaped cutting edge.

6. Tool according to claim 5, characterized in that the shaft comprises at least a lengthwise passage and lateral openings.

7. Tool according to one of claims 1 to 6, characterized by means (7, 8, 9, 10, 11; 20, 21) for the admission of a rinsing fluid.

8. Tool according to one of claims 1 to 7, characterized by means (12, 14, 15, 16, 17; 24) for the evacuation of machining residues.

9. Tool according to claims 3, 7 and 8, or according to claims 6, 7 and 8, characterized in that the admission means are shaped for delivering rinsing fluid to the interior of the hollow body, respectively of the shaft, and that the evacuation means are shaped for removing the rinsing fluid contaminated with machining residues from the interior of the hollow body, respectively the shaft.

10. Tool according to one of the claims 1 to 9, characterized in that the tool is sterile and enclosed in a wrapping which maintains its sterile state.

## Revendications

1. Outil (1) pour le façonnage des os, avec un organe de façonnage (2) comportant au moins un tranchant (4) pour enlever des parties d'os par rotation de l'outil, et avec un adaptateur (3) pour accoupler l'outil (1) à un moyen d'entraînement pouvant être mis en rotation, caractérisé en ce que l'organe de façonnage (2) ainsi qu'au moins son unique tranchant (4) sont en matière plastique.

2. Outil selon la revendication 1, caractérisé en ce que l'adaptateur (3) est en matière plastique.

3. Outil selon une des revendications 1 ou 2, caractérisé en ce que l'organe de façonnage (2) est un corps creux dont la face extérieure comporte plusieurs tranchants (4) et des ouvertures (5, 6).

4. Outil selon une des revendications 1 ou 2, caractérisé en ce que l'organe de façonnage (2) est un corps plein dont la face extérieure comporte plusieurs tranchants (4).

5. Outil selon une des revendications 1 ou 2, caractérisé en ce que l'organe de façonnage comporte une tige allongée autour de laquelle court au moins un tranchant en spirale.

6. Outil selon la revendication 5, caractérisé en ce que la tige comporte au moins un canal longitudinal et des ouvertures latérales.

7. Outil selon une des revendications 1 à 6, caractérisé par des moyens d'amenée (7, 8, 9, 10, 11; 20, 21) pour un liquide de rinçage.

8. Outil selon une des revendications 1 à 7, caractérisé par des moyens d'évacuation (12, 14, 15, 16, 17; 24) pour l'évacuation des résidus du façonnage.

9. Outil selon les revendications 3, 7 et 8, ou selon les revendications 6, 7 et 8, caractérisé en ce que les moyens d'amenée sont agencés pour alimenter l'intérieur du corps creux, respectivement de la tige, en liquide de rinçage, et que les moyens d'évacuation sont agencés pour évacuer le liquide de rinçage souillé par les résidus du façonnage hors du corps creux, respectivement hors de la tige.

10. Outil selon une des revendications 1 à 9, caractérisé en ce que l'outil de façonnage est stérile et empaqueté dans une enveloppe qui maintient cet état stérile.
